# EUROPEAN PATENT APPLICATION

(11) **EP 1 723 918 A1**
(43) Date of publication of application: **22.11.2006**
(21) Application number: 06010069.0
(22) Date of filing: 16.05.2006
(51) Int. Cl.: A61B 17/34

(54) **Laparoscope supporting device**

(30) Priority: 16.05.2005 JP 2005143020
(71) Applicant: Fujinon Corporation, Saitama-shi, Saitama (JP)
(72) Inventor: Igarashi, Tatsuo, Chiba-shi Chiba (JP); Miyake, Yoichi, Sakura-shi Chiba (JP); Nakaguchi, Toshiya, Funabashi-shi Chiba (JP); Makino, Harufumi, Chiba-shi Chiba (JP); Fujita, Hiroshi, Saitama-shi Saitama (JP); Norinobu, Tomoya, Saitama-shi Saitama (JP)
(74) Representative: Klunker . Schmitt-Nilson . Hirsch

(57) **Abstract**

A laparoscope supporting device which is attached to a body wall face of an examinee and supports a laparoscope to be inserted in a body cavity of the examinee by using an inserting tool as a guide, the device comprises: a grasping portion that grasps the inserting tool; and a support portion that supports the grasping portion in a movable manner in a predetermined direction.

## Description

### Background of the Invention

### 1. Field of the Invention

The present invention relates to a laparoscope supporting device, and in particular, to a laparoscope supporting device for supporting a laparoscope to be inserted in a body cavity of an examinee via an inserting tool in a desired posture.

### 2. Description of the Related Art

A laparoscope as an internal body cavity observation apparatus is a device for applying various treatments such as a cure of adhesion by puncturing a body cavity with a trocar (inserting tool) from the surface of the abdominal region of an examinee, and then inserting the inserting portion of a laparoscope into the body cavity by using this trocar as a guide while displaying whether an adhesion occurs in the body cavity and objects to be observed such as an ovarian tumor or uterine myoma on a monitor (for example, JP-A-2003-265402).

At a time of clinical operations with a laparoscope, normally, three or four holes are opened in the abdominal region and a plurality of treatment tools are inserted from these holes via a trocar, respectively, whereby treatment is applied. An operator who operates the treatment tools operates the treatment tools while looking at a video image of an object to be observed on a monitor obtained through the laparoscope. Therefore, according to instructions from the operator who operates the treatment tools, the posture of the laparoscope is changed for each instruction, and the portion of the observation object to be imaged is changed. Namely, an operator who holds the laparoscope and changes the posture of the laparoscope and one or two operators who assist the whole of the clinical operations take part in the clinical operations with the laparoscope in addition to one or two operators who operate the treatment tool.

However, operators who take part in the clinical operations by using the related-art laparoscope are as many as 4 or 5 operators, and it has been demanded to reduce the number of operators.

### Summary of the Invention

The present invention was developed in view of these circumstances, and an object thereof is to provide a laparoscope supporting device which can reduce the number of operators who take part in clinical operations using a laparoscope.

To achieve the above-described object, according to the invention set forth in a first aspect of the invention, a laparoscope supporting device which is attached to a body wall face of an examinee and supports a laparoscope to be inserted in a body cavity of the examinee by using an inserting tool as a guide, includes: a grasping portion for grasping the inserting tool, and a support portion for supporting the grasping portion in a movable manner in a predetermined direction.

According to the invention set forth in the first aspect of the invention, the laparoscope supporting device is attached to a body wall face of an examinee, the inserting tool is grasped by the grasping portion of the laparoscope supporting device, and the laparoscope is inserted into a body cavity by using this inserting tool as a guide. The laparoscope is moved to a position that the operator desires by a support portion that supports the grasping portion movably in a predetermined direction, and supported in this posture. Therefore, by using the laparoscope supporting device of the invention, an operator exclusively for holding the laparoscope and changing the position of the laparoscope becomes unnecessary, so that the number of operators can be reduced.

According to the invention set forth in a second aspect of the invention, in the laparoscope supporting device according to the first aspect of the invention, the support portion includes: a turning support portion that supports the grasping portion in a manner enabling it to turn on the body wall face of the examinee, and a tilting support portion that supports the grasping portion in a manner enabling it to tilt with respect to the body wall face of the examinee.

According to the invention set forth in the second aspect of the invention, by turning the grasping portion by the turning support portion and tilting the grasping portion by the tilting support portion, the laparoscope grasped by the grasping portion can be changed into various postures.

According to the invention set forth in a third aspect of the invention, in the laparoscope supporting device according to the second aspect of the invention, the turning support portion has a turning fixing portion that fixes the grasping portion at a desired turning position, and the tilting support portion has a tilt fixing portion that fixes the grasping portion at a desired tilt position.

According to the invention set forth in the third aspect of the invention, the grasping portion can be fixed at a desired turning position by the turning fixing portion, and the grasping portion can be fixed at a desired tilt position by the tilt fixing portion, so that the laparoscope can be fixed in a desired posture.

According to the invention set forth in a fourth aspect of the invention, in the laparoscope supporting device according to the second or third aspect of the invention, the center of turning of the grasping portion by the turning support portion and the center of tilting of the grasping portion by the tilting support portion are matched with each other, and these centers are set and positioned on the body wall face of the examinee.

According to the invention set forth in the fourth aspect of the invention, by matching the center of turning of the grasping portion by the turning support portion with the center of tilting of the grasping portion by the tilting support portion, and setting and positioning these centers on the body wall face of the examinee, the laparoscope turns and tilts around a pivot of one point on the body wall face, so that the size of an opening for inserting the inserting tool to be opened in the body wall of the examinee can be minimized.

### Brief Description of the Drawings

Fig. 1 is a system construction diagram of an internal body cavity observation apparatus including a laparoscope supporting device;
Fig. 2 is an explanatory view showing a clinical situation while using the laparoscope supporting device shown in Fig. 1;
Fig. 3 is an external view of the clinical situation of Fig. 2;
Fig. 4 is an entire perspective view of the laparoscope supporting device;
Fig. 5 is an assembly perspective view of the turning support portion of the laparoscope supporting device;
Fig. 6 is a perspective view of a state in that the ratchet claws are engaged with the ratchet teeth;
Fig. 7 is an entire perspective view of the turning support portion;
Fig. 8 is an entire perspective view of the tilting support portion;
Fig. 9 is an exploded view of the tilting support portion;
Fig. 10 is an exploded view of the tilting support portion; and
Fig. 11 is an enlarged perspective view showing the construction of the knob.

### Detailed Description of the Invention

Hereinafter, a preferred embodiment of the laparoscope supporting device of the invention will be explained with reference to the accompanying drawings.

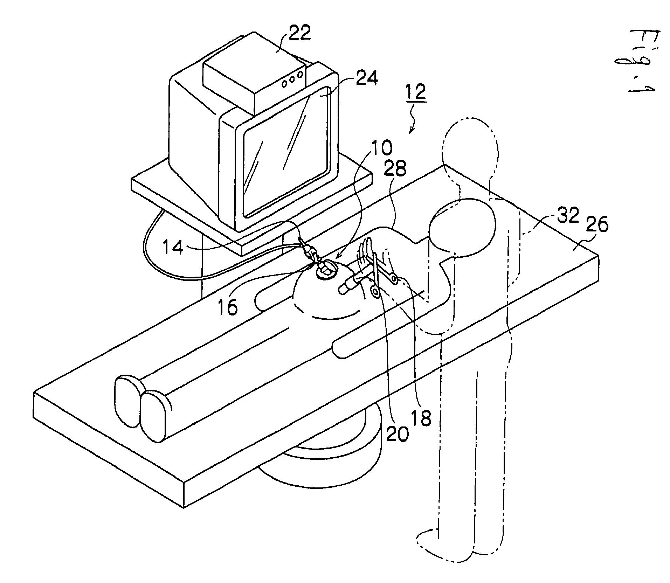

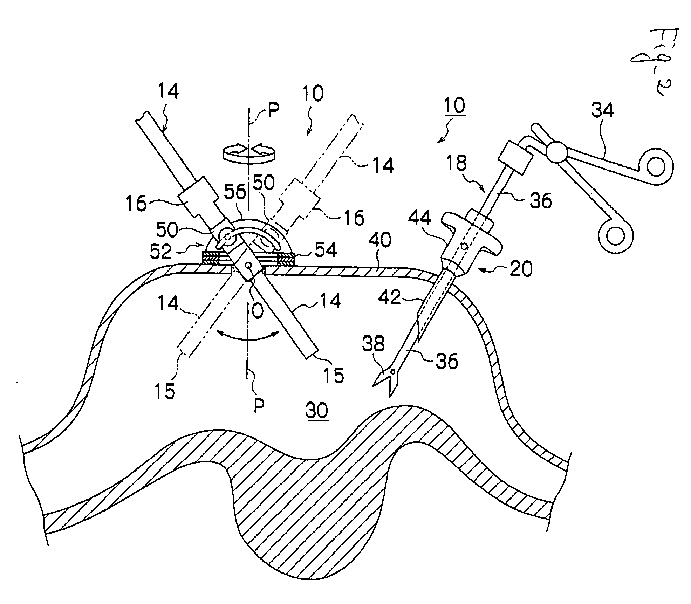

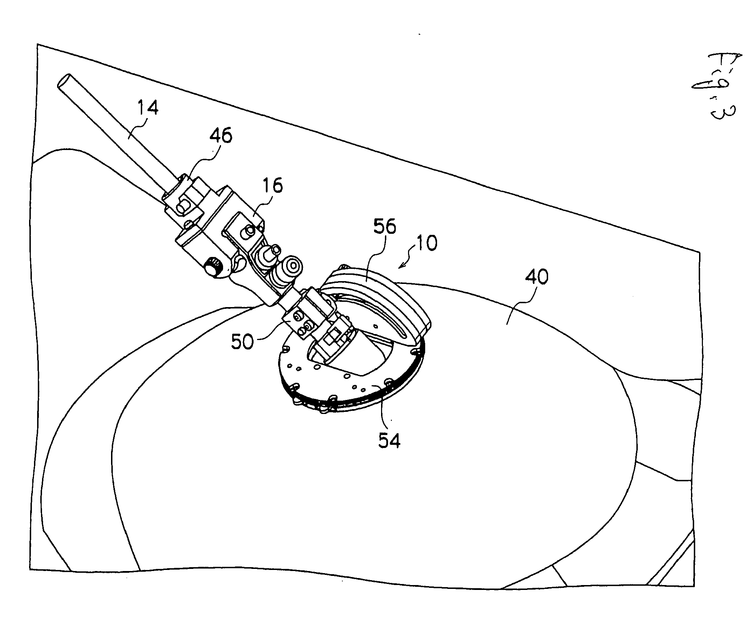

Fig. 1 is a system construction diagram of an internal body cavity observation apparatus 12 including a laparoscope supporting device 10 according to the embodiment, Fig. 2 is a sectional view of a clinical situation while using the laparoscope supporting device 10, and Fig. 3 is an external view of the clinical situation while using the laparoscope supporting device 10.

As shown in Fig. 1 through Fig. 3, the internal body cavity observation apparatus 12 includes a laparoscope supporting device 10, a laparoscope 14, a laparoscope trocar (inserting tool) 16, a treatment tool 18, a treatment tool trocar 20, a video signal processor 22, a monitor 24, and so on.

The treatment tool 18 is for treatment of a diseased portion inside a body cavity 30 of an examinee 28 laid on an operating table 26, and is provided with an operating portion 34 to be operated by an operator 32. In this treatment tool 18, the inserting portion 36 is connected to the front end of the operating portion 34, and at the front end of the inserting portion 36, a forceps portion 38 is provided.

The inserting portion 36 of the treatment tool 18 is inserted into the body cavity 30 by using the treatment tool trocar 20 puncturing the abdominal region 40 of the examinee from the surface as shown in Fig. 2. The trocar 20 includes a metallic hollow tube 42 formed so as to have a sharp-pointed front end, and a soft grasping tube 44 provided on the base end of the hollow tube 42. The hollow tube 42 of the trocar 20 thus constructed is inserted into the body cavity 30 when an operator 32 grasps the grasping tube 44 and punctures the abdominal region 40 with the sharp-pointed front end set as the insertion leading end of the hollow tube 42.

The laparoscope trocar 16 is a guide member for inserting the laparoscope 14 into the body cavity 30, and on its base portion, a fixing member 46 for fixing the laparoscope 14 inserted in the laparoscope trocar 16 to the laparoscope trocar 16 is provided as shown in Fig. 3.

The laparoscope supporting device 10 is a device for supporting the laparoscope 14 including an observation optical system and an illumination system installed in its front end 15 at the abdominal region 40 of the examinee 28 and turning the front end 15 to a desired direction and supporting it at this position.

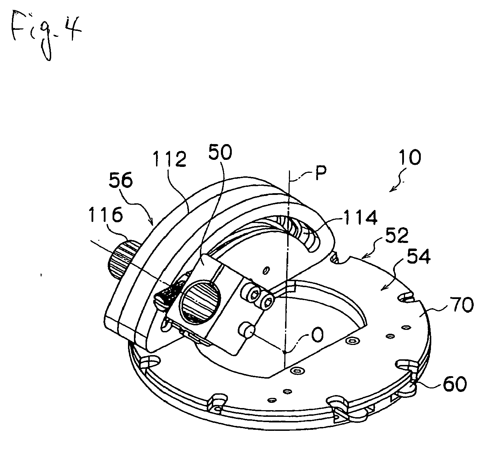

The laparoscope supporting device 10 mainly includes a grasping portion 50 for grasping the laparoscope trocar 16, and a movement support portion (support portion) 52 for supporting the grasping portion 50 movably in a predetermined direction as shown in Fig. 3 and Fig. 4.

The movement support portion 52 includes a turning support portion 54 and a tilting support portion 56 as shown in Fig. 2, and the turning support portion 54 supports the grasping portion 50 in a manner enabling it to turn around a pivot P orthogonal to the skin surface of the abdominal region 40 of the examinee 28, and the tilting support portion 56 supports the graphing portion 50 in a manner enabling it to tilt with respect to the pivot P.

Furthermore, the center O of turning of the grasping portion 50 by the turning support portion 54 and the center O of tilting of the grasping portion 50 by the tilting support portion 56 are matched with each other, and the centers O are set and positioned on the skin surface of the abdominal region 40 of the examinee 28.

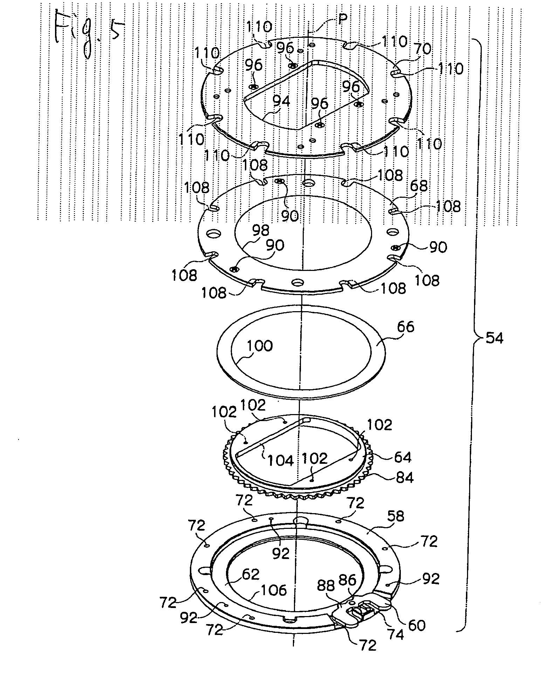

The turning support portion 54 includes, as shown in Fig. 5, a turning seat 58, a lever 60, a sliding member 62, a gear 64, a sliding member 66, an intermediate ring 68, and a rotating ring 70, and the members except for the lever 60 are roughly formed into donut shapes having openings at their centers.

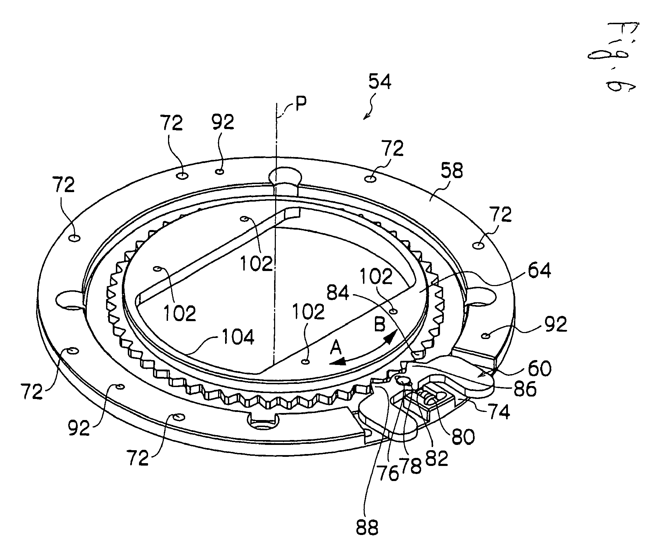

The turning seat 58 has pores 72, 72... formed at predetermined positions of the outer circumferential surface as shown in Fig. 6, and is fixed to the skin surface of the abdominal region 40 of the examinee 28 by a suture stitched via these pores 72, 72....

In the outer circumference of the turning seat 58, a counter-bored notch 74 is made, and to this notch 74, the lever 60 is attached via a pin 76 so as to freely swing. The pin 76 is inserted in a elongate hole 78 formed at the center of the lever 60, and thereby, the lever 60 is attached to the turning seat 58 so as to freely advance to and withdraw from the gear 64 disposed on the inner side of the turning seat 58. The lever 60 is pressed in a direction to advance to the gear 64 by a pressing force of a spring 80 provided in the notch 74 of the turning seat 58. The reference numeral 82 denotes a steel ball for transmitting the pressing force of the spring 80 to the lever 60.

On the other hand, the lever 60 has an operating portion formed in a two-forked shape, and on the inner side thereof, two ratchet claws (turning fixing portions) 86 and 88 to be engaged with ratchet teeth (turning fixing portions) 84 of the gear 64 are provided so as to project. When the ratchet claws 8 6 are engaged with the ratchet teeth 84 by a swing switch operation on the lever 60 by the operating portion, the gear 64 is allowed to rotate only in the arrowA direction of Fig. 6, and when the ratchet claws 88 are engaged with the ratchet teeth 84, the gear 64 is allowed to rotate only in the arrowB direction of Fig. 6. The gear 64 is attached to the turning seat 58 in a rotatable manner by being clipped between the sliding member 62 fixed to the inner circumferential flange portion (not shown) of the turning seat 58 and the sliding member 66 interposed between the gear 64 and the intermediate ring 68.

The intermediate ring 68 is a ring for pressing the sliding member 66 against the gear 64, and is fixed to the turning seat 58 by screwing screws 90, 90... attached to the intermediate ring 68 into screw holes 92, 92... of the turning seat 58.

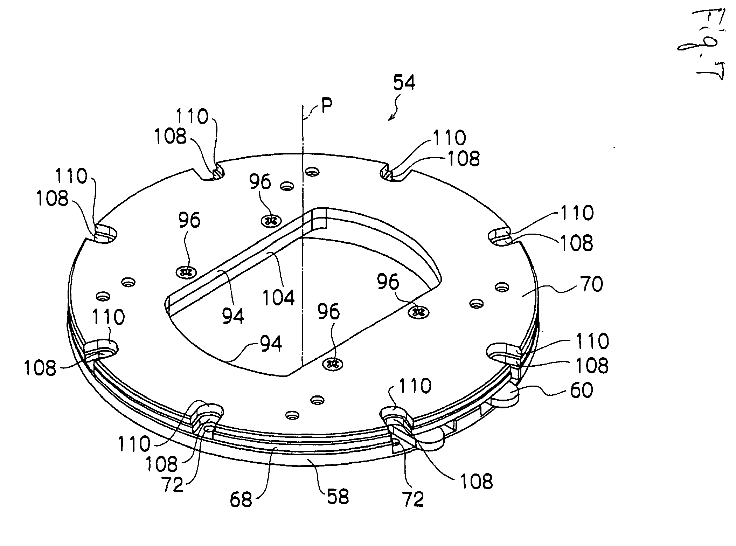

The rotating ring 70 is slidably supported on the intermediate ring 68. The rotating ring 70 is fixed to the gear 64 by screwing the screws 96, 96... attached around the opening 94 of the rotating ring 70 into the screw holes 102, 102... of the gear 64 via the opening 98 of the intermediate ring 68 and the opening 100 of the sliding member 66. Therefore, the rotating ring 70 can rotate together with the gear 64, and by engaging either the claws 86 or claws 88 with the ratchet teeth 84 or a selecting operation of the lever 60, the rotating ring is allowed to rotate in any one direction. Thereby, the turning support portion 54 is assembled as shown in Fig. 7.

Then, the laparoscope trocar 16 is inserted via the opening 94 of the rotating ring 70, the opening 98 of the intermediate ring 68 (see Fig. 5), the opening 100 of the sliding member 66, the opening 104 of the gear 64, the opening 106 of the sliding member 62, and an unillustrated opening of the turning seat 58. In the circumferential edge of the intermediate ring 68, arched notches 108, 108... are formed at positions corresponding to the pores 72 of the turning seat 58, and in the circumferential edge of the rotating ring 70, arched notches 110, 110... are also formed at positions and by the number corresponding to the notches 108 and 108 of the intermediate ring 68. Therefore, when the turning seat 58 is fixed to the skin surface of the abdominal region 40 of the examinee 28 by a suture, the intermediate ring 68 and the rotating ring 70 are rotated as appropriate, and as shown in Fig. 7, the notches 108 and 110 are matched with each other so that the pores 72, 72... are exposed as shown in Fig. 7.

The tilting support portion 56 is fixed to the rotating ring 70 of the turning support portion 54 by unillustrated screws as shown in Fig. 4. Therefore, the tilting support portion 56 is rotatable around the pivot P together with the rotating ring 70.

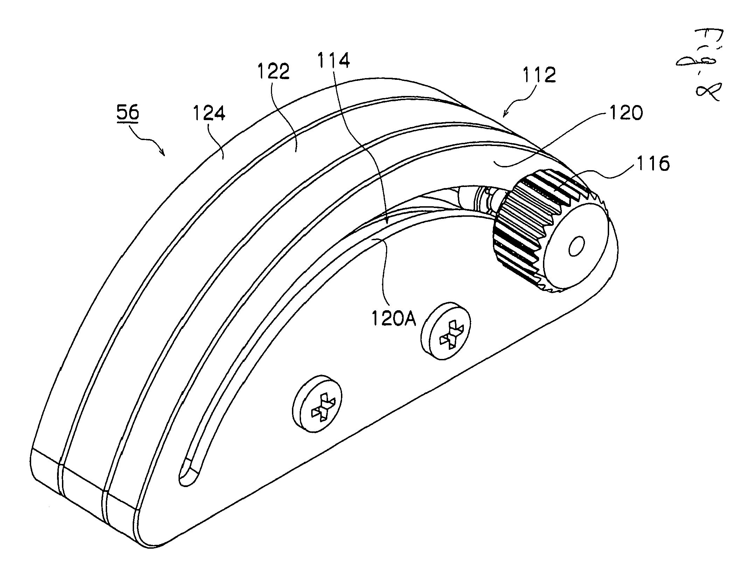

This tilting support portion 56 has a guide portion 112 roughly formed into a semicircular shape as shown in Fig. 4 and Fig. 8, and the guide groove 114 that tilts the grasping portion 50 around the center O of tilting is formed so as to penetrate the guide portion 112.

In the guide groove 114, the shaft of a knob (tilt fixing portion) 116 that tilts the grasping portion 50 along the guide groove 114 and fixes the grasping portion 50 at a desired tilt position is slidably engaged.

The guide portion 112 includes, as shown in Fig. 8 through Fig. 10, an outer plate 120, a middle plate 122, and an inner plate 124, and the plates 120, 122, and 124 are overlapped and fixed so that grooves 120A, 122A, and 124A formed therein are matched with each other. By matching the grooves 120A, 122A, and 124A with each other, the guide groove 114 is formed to form the tilting support portion 56 shown in Fig. 8.

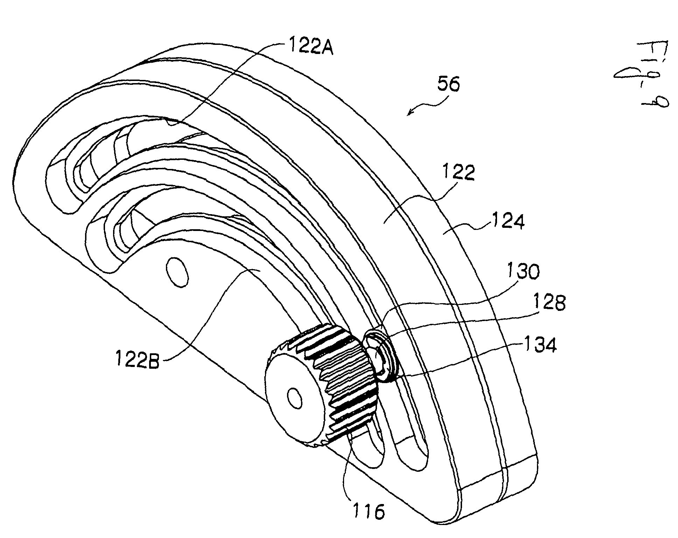

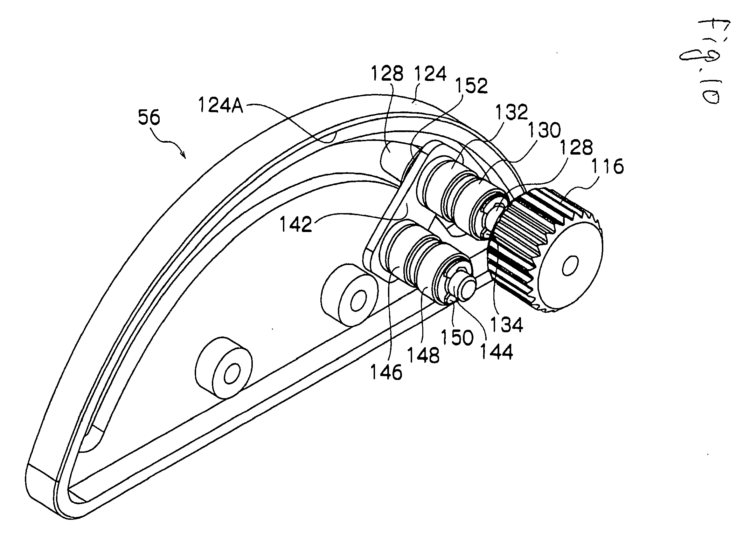

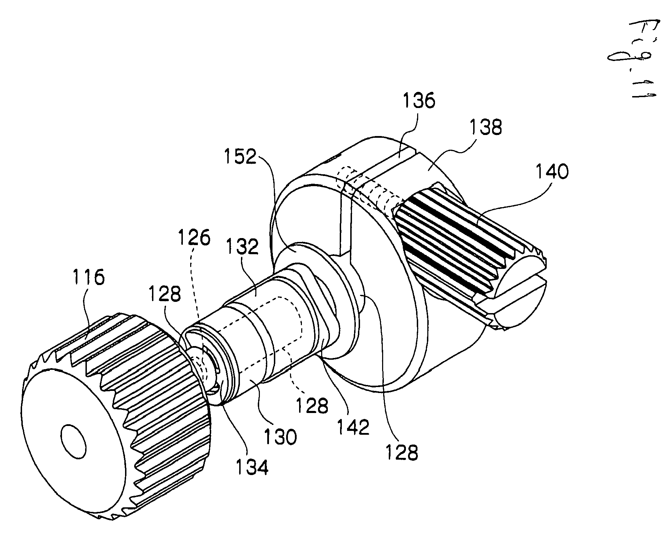

As shown in Figs. 10 and 11, the knob 116 is joined to a shaft 128 by screwing a male screw 126 provided coaxially with the knob 116 into the base end of the shaft 128. Through the shaft 128, bearings 130 and 132 are fitted rotatably, and the bearings 130 and 132 are prevented from deviating from the shaft 128 by an E-shaped stopper ring 134. The bearing 130 is fitted in the guide groove 122A formed in the middle plate 122 as shown in Fig. 9, and thereby, the shaft 128 and the knob 116 are made movable along the groove 114.

The front end of the shaft 128 is stuck out from the groove 124A of the inner plate 124, and to the front end, a joint 138 having a slot 136 formed as shown in Fig. 11 is joined. To this joint 138, the shaft (not shown) of the grasping portion 50 shown in Fig. 4 is joined, and by screwing a screw 140 of the joint 138, the grasping portion 50 is fixed to the joint 138. Thereby, the grasping portion 50 is joined to the knob 116 via the joint 138 and the shaft 128.

To the shaft 128, as shown in Fig. 10, the upper portion of a rectangular bracket 142 is fixed as shown in Fig. 10, and to the lower portion of the bracket 142, a shaft 144 extending parallel to and in the same direction as the shaft 128 is fixed. Through this shaft 144, bearings 146 and 148 are fitted rotatably, and the bearings 146 and 148 are prevented from deviating from the shaft 144 by an E-shaped stopper ring 150. The bearing 148 is slidably fitted in the guide groove 122B of the middle plate 122 shown in Fig. 9.

The guide groove 122B is formed around the center O of tilting (see Fig. 2) according to the groove 122A on the inner side of the groove 122A. Therefore, the knob 116 is moved around the center O of tilting along the groove 122A and the guide groove 122B. At this time, the shaft 128 moves along the groove 122A, while its rotation around its own axis is prevented, by moving the shaft 144 fixed to the bracket 142 along the guide groove 122B. Therefore, the grasping portion 50 to be joined to the shaft 128 via the joint 138 (Fig 11) is moved around the center O of tilting while its rotation around its own axis is prevented, so that the laparoscope 14 supported on the grasping portion 50 via the laparoscope trocar 16 is tilted around the center O of tilting as shown in Fig. 2.

To the shaft 128, a disk-shaped pressure clipping plate 152 is fixed. By screwing the knob 116 to the shaft 128 via the male screw 126, the knob 116 and the pressure clipping plate 152 can pressure-clip and hold the guide portion 112. Thereby, the movement of the knob 116 is locked, so that the laparoscope 14 is fixed at a desired tilt position.

Next, action of the laparoscope supporting device 10 will be explained.

The laparoscope supporting device 10 is fixed to the skin surface of the abdominal region 40 of the examinee 28 by a suture as shown in Fig. 3, and at this position, the laparoscope trocar 16 puncturing the abdominal region 40 from the surface is grasped by the grasping portion 50 of the laparoscope supporting device 10. Next, the laparoscope 14 is inserted by using the laparoscope trocar 16 as a guide, and when the front end 15 of the laparoscope 14 is sufficiently inserted into the body cavity 30 as shown in Fig. 2, the laparoscope 14 is fixed to the laparoscope trocar 16 by the fixing member 46 of the laparoscope trocar 16.

Thereafter, as shown in Fig. 1, a treatment using the laparoscope 14 is started. At this time, for example, when the operator 32 wants to observe a diseased portion inside the body cavity 30, the operator 32 operates the turning support portion 54 to rotate the grasping portion 50 around the pivot P, and operates the tilting support portion 56 to tilt the grasping portion 50 with respect to the pivot P, whereby changing the posture of the laparoscope 14 to turn the front end 15 of the laparoscope 14 toward the diseased portion. Thereby, an observation video image of the diseased portion is displayed on the monitor 24.

Then, when the operator wants to lock the posture of this laparoscope 14, he/she screws-in the knob 116 of the tilting support portion 56 to lock the movement of the grasping portion 50 in the tilting direction. Thereby, the posture of the laparoscope 14 in the tilting direction is fixed. Regarding the posture in the turning direction of the laparoscope 14, the ratchet claws 86 or claws 88 are engaged with the ratchet teeth 84 of the turning support portion 54, so that the laparoscope 14 is fixed in this posture in the turning direction. The operator performs treatment of the diseased portion by operating the treatment tool 18 while observing the video image of the diseased portion displayed on the monitor 24 by the laparoscope 14. Thereafter, when the operator wants to change the posture of the laparoscope 14, as described above, he/she operates the turning support portion 54 and the tilting support portion 56 to change the posture of the laparoscope 14 into a desired posture.

Thus, according to the laparoscope supporting device 10 of the embodiment, the operator 32 can turn the laparoscope 14 toward various desired directions by operating the turning support portion 54 and the tilting support portion 56, and can fix the laparoscope 14 in the desired posture, so that an operator exclusively for holding the laparoscope 14 and changing the position of the laparoscope 14 becomes unnecessary. Therefore, by using this laparoscope supporting device 10, the number of operators can be reduced.

According to the laparoscope supporting device 10, the center O of turning of the grasping portion 50 by the turning support portion 54 and the center O of tilting of the grasping portion 50 by the tilting support portion 56 are matched with each other, and these centers O are positioned on the body wall face of the examinee 28, so that the laparoscope 14 turns and tilts around a pivot of one point on the body wall face. Therefore, the size of the puncture opening for the laparoscope trocar 16 to be opened in the body wall of the examinee 28 can be minimized.

The laparoscope supporting device 10 of the embodiment is an example of manual changing of the posture of the laparoscope 14, however, it is also allowed to electrically change the posture of the laparoscope 14. Namely, motors are attached to the respective turning support portion 54 and tilting support portion 56, and by driving forces of these motors, the grasping portion 50 is turned and tilted by driving forces of these motors, whereby the posture of the laparoscope 14 is electrically changed. ON/OFF operations of these motors can be made by the operator 32 by a foot switch provided under the operating table 26, or can be made by another operator by operating an operation member such as a joystick provided in an operator room.

According to the laparoscope supporting device of the invention, the laparoscope supporting device is attached to the body wall face of an examinee, an inserting tool is grasped by the grasping portion of this laparoscope supporting device, and the laparoscope is positioned in a posture that an operator desires by a support portion that supports the grasping portion movably in a predetermined direction, so that an operator exclusively for holding the laparoscope and changing the position of the laparoscope becomes unnecessary. Therefore, the invention reduces the number of operators.

The entire disclosure of each and every foreign patent application from which the benefit of foreign priority has been claimed in the present application is incorporated herein by reference, as if fully set forth.

## Claims

1. A laparoscope supporting device which is attached to a body wall face of an examinee and supports a laparoscope to be inserted in a body cavity of the examinee by using an inserting tool as a guide, the device comprising:
a grasping portion that grasps the inserting tool; and
a support portion that supports the grasping portion in a movable manner in a predetermined direction.

2. The laparoscope supporting device according to Claim 1, wherein the support portion comprises:
a turning support portion that supports the grasping portion in a manner enabling it to turn on the body wall face of the examinee; and
a tilting support portion that supports the grasping portion in a manner enabling it to tilt with respect to the body wall face of the examinee.

3. The laparoscope supporting device according to Claim 2,
wherein the turning support portion comprises a turning fixing portion that fixes the grasping portion at a desired turning position, and
the tilting support portion comprises a tilt fixing portion that fixes the grasping portion at a desired tilt position.

4. The laparoscope supporting device according to Claim 2,
wherein a center of turning of the grasping portion by the turning support portion and a center of tilting of the grasping portion by the tilting support portion are matched with each other, and the centers are set and positioned on the body wall face of the examinee.

5. The laparoscope supporting device according to Claim 3,
wherein a center of turning of the grasping portion by the turning support portion and a center of tilting of the grasping portion by the tilting support portion are matched with each other, and the centers are set and positioned on the body wall face of the examinee.
